# EUROPEAN PATENT APPLICATION

(11) **EP 3 178 370 A1**
(43) Date of publication of application: **14.06.2017**
(21) Application number: 15829851.3
(22) Date of filing: 26.03.2015
(51) Int. Cl.: A61B 1/00

(54) **ANTENNA RECEIVING DEVICE, ANTENNA HOLDER, AND RECEIVING DEVICE**

(30) Priority: 08.08.2014 JP 2014162363
(71) Applicant: Olympus Corporation, Tokyo 192-8507 (JP)
(72) Inventor: TANAKA, Shinsuke, Hachioji-shi Tokyo 192-8507 (JP)
(74) Representative: Gunzelmann, Rainer
(86) International application number: PCT/JP2015/059385
(87) International publication number: WO 2016/021229

(57) **Abstract**

There is provided an antenna receiving device, an antenna holder, and a receiving device that allow receiving antennas to be easily adjusted to predetermined receiving positions on the antenna holder regardless of a body shape of a subject. The antenna receiving device includes: a belt-shaped sheet portion 51 to which a plurality of receiving antennas 4 is detachably connected to receive information transmitted from a body-insertable apparatus and that is wrapped around and attached on a trunk of the subject; a shape detection unit 52 that is provided on the outer surface of the sheet portion 51 along a longitudinal direction of the sheet portion 51 to detect the shape of the sheet portion 51; an estimation unit 651 that estimates a cross-sectional shape of the trunk of the subject; a position defining unit 652 that defines a plurality of indexes M1 corresponding to attachment positions of the plurality of receiving antennas 4 on the sheet portion 51 based on the cross-sectional shape estimated by the estimation unit 651; and a display unit 63 that displays index information indicating the plurality of indexes M1 defined by the position defining unit 652.

## Description

### Field

The present invention relates to an antenna receiving device for receiving information from a body-insertable apparatus that is configured to be introduced into a subject and move in a body cavity of the subject to acquire information on the subject. The present invention also relates to an antenna holder and a receiving device. Background

In the field of endoscopes, there has been conventionally known a capsule endoscope in which an imaging function, a wireless communication function, and others are built in a capsule-shaped casing that is small enough to be introduced into a digestive tract of a subject such as a patent. After being swallowed by the subject, the capsule endoscope sequentially captures images of the inside of the subject while moving inside the subject such as the digestive tract by peristaltic movement or the like to generate image data, and sequentially transmits the image data wirelessly.

The image data transmitted wirelessly by the capsule endoscope as described above is received by a receiving device via a plurality of receiving antennas provided outside the subject. Each of the receiving antennas is inserted into a viscous antenna holder and stuck to the body surface of the subject. The sticking positions of the receiving antennas are decided with an index such as the subject's navel or costa. This requires the adjustment of the sticking positions for each subject, which takes excessive time and trouble to medical personnel. As one of techniques for alleviating the workload of sticking, there is known a technique by which a cloth antenna holder is wound around the subject and receiving antennas are attached to the outer surface of the antenna holder at a plurality of specified positions (refer to Patent Literature 1).

### Citation List

### Patent Literature

Patent Literature 1: Japanese Patent No. 5193402 Summary

### Technical Problem

However, according to the technique described in Patent Literature 1, the antenna holder deforms depending on the body shape of the subject, and the positions of the receiving antennas are displaced on the body surface. It is thus difficult to attach the plurality of receiving antennas at their appropriate positions.

The present invention has been made in view of the foregoing, and an object of the present invention is to provide an antenna receiving device, an antenna holder, and a receiving device that allow a plurality of receiving antennas to be easily attached to an outer surface of the antenna holder at predetermined positions regardless of a body shape of a subject.

### Solution to Problem

In order to solve the above described problem and achieve the object, an antenna receiving device according to the invention includes: a belt-shaped sheet portion to which a plurality of receiving antennas is detachably attached to receive information transmitted from a body-insertable apparatus introduced into a subject to acquire in-vivo information of the subject, the sheet portion being configured to be wrapped around and attached on a trunk of the subject; a plurality of indexes provided on an outer surface of the sheet portion to indicate positions on the outer surface; shape detection units provided on the outer surface of the sheet portion along a longitudinal direction of the sheet portion to detect a shape of the sheet portion; an estimation unit configured to estimate a cross-sectional shape of the trunk based on the shape of the sheet portion detected by the shape detection units; a position defining unit configured to define the plurality of indexes corresponding to attachment positions of the plurality of receiving antennas on the outer surface of the sheet portion based on the cross-sectional shape estimated by the estimation unit; and a display unit configured to display index information indicating the plurality of indexes defined by the position defining unit.

In the antenna receiving device according to the above-described invention, the shape detection units are respectively provided at least near edge portions in a width direction of the sheet portion.

In the antenna receiving device according to the above-described invention, the plurality of indexes is coordinates indicating the positions on the outer surface of the sheet portion, and the display unit is configured to display the index information by the coordinates.

In the antenna receiving device according to the above-described invention, the outer surface of the sheet portion is divided into a plurality of areas at predetermined intervals such that different colors are applied to the areas as the indexes, and the display unit is configured to display the index information by the colors.

In the antenna receiving device according to the above-described invention, the plurality of indexes is light-emitting units configured to emit light of predetermined colors, and the display unit is configured to display the plurality of indexes by the colors of the light emitted from the light-emitting units.

An antenna holder according to the invention is configured to be connected to a receiving device for displaying images corresponding to information that is transmitted from a body-insertable apparatus introduced into a subject to acquire in-vivo information of the subject and that is received by a plurality of receiving antennas. The antenna holder includes: a belt-shaped sheet portion to which the plurality of receiving antennas is detachably attached, the sheet portion being configured to be wrapped around and attached on a trunk of the subject; a plurality of indexes provided on an outer surface of the sheet portion to indicate positions on the outer surface; and a shape detection unit provided on the outer surface of the sheet portion along a longitudinal direction of the sheet portion to detect a shape of the sheet portion.

A receiving device according to the invention is configured to display information that is transmitted from a body-insertable apparatus introduced into a subject to acquire in-vivo information of the subject and that is received by a plurality of receiving antennas. The receiving device includes: an estimation unit configured to estimate a cross-sectional shape of a trunk of the subject based on a shape of a belt-shaped sheet portion wrapped around and attached on the trunk of the subject, the shape of the sheet portion has been detected by a shape detection unit provided along a longitudinal direction of the sheet portion; a position defining unit configured to define, based on the cross-sectional shape estimated by the estimation unit, a plurality of indexes provided on an outer surface of the sheet portion to indicate positions on the sheet portion, the plurality of indexes corresponding to attachment positions of the plurality of receiving antennas on the outer surface of the sheet portion; and a display unit configured to display index information indicating the plurality of indexes defined by the position defining unit.

### Advantageous Effects of Invention

According to the present invention, it is possible to easily attach the plurality of receiving antennas on the antenna holder at predetermined positions regardless of a body shape of the subject.

### Brief Description of Drawings

FIG. 1 is a schematic diagram illustrating a general configuration of a capsule endoscope system according to an embodiment of the present invention.
FIG. 2 is a perspective view of a general configuration of an antenna holder illustrated in FIG. 1.
FIG. 3 is an enlarged view of a region D illustrated in FIG. 2.
FIG. 4 is a block diagram illustrating a functional configuration of a receiving device illustrated in FIG. 1.
FIG. 5A is a diagram illustrating an example of a cross-sectional shape of body surface of a subject.
FIG. 5B is a diagram illustrating an example of a cross-sectional shape of body surface of another subject.
FIG. 6 is a diagram illustrating an example of an image displayed on a display unit of the receiving device illustrated in FIG. 1.
FIG. 7 is a schematic view of a configuration of a light-emitting unit according to a second modified example of the embodiment of the present invention.

### Description of Embodiments

Modes carrying out the present invention will be explained below in detail with reference to the drawings. However, the present invention is not limited to embodiments. The drawings referred to in the following explanation illustrate schematically shapes, sizes, and positional relationships to a degree that the contents of the present invention can be understood. That is, the present invention is not limited to the shapes, sizes, and positional relationships illustrated in the drawings. In addition, the following explanation will be given as to a capsule endoscope system including an antenna receiving device that receives wireless signals from a body-insertable apparatus (hereinafter, referred to as "capsule endoscope") configured to be introduced into the body of the subject to capture in-vivo images of the subject as an example. However, the present invention is not limited to the embodiment. The same reference signs are used to designate the same elements throughout the drawings.

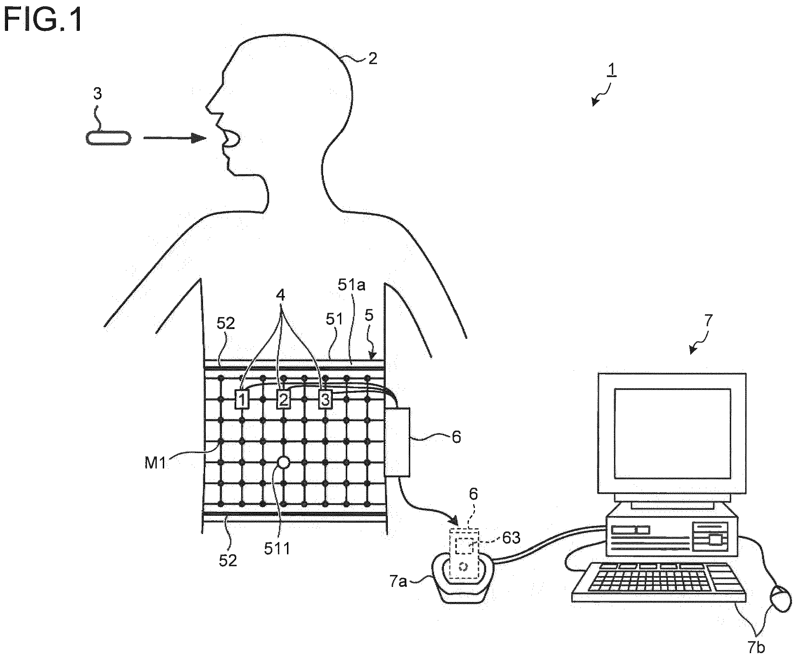

FIG. 1 is a schematic diagram illustrating a general configuration of a capsule endoscope system according to the embodiment of the present invention.

A capsule endoscope system 1 illustrated in FIG. 1 includes: a capsule endoscope 3 that captures in-vivo images of a subject 2; a plurality of receiving antennas 4 that receives information transmitted from the capsule endoscope 3 introduced into the subject 2; an antenna holder 5 to which the plurality of receiving antennas 4 are detachably connected and that is wrapped around the trunk of the subject 2; a receiving device 6 that performs predetermined processing on wireless signals received by the receiving antennas 4 and records or displays the results; and an image processing device 7 that processes and/or displays images corresponding to image data of the subject 2 captured by the capsule endoscope 3. In the first embodiment, the antenna holder 5 and the receiving device 6 serve as an antenna receiving device.

The capsule endoscope 3 has an imaging function that captures images of inside of the subject 2 and a wireless communication function that transmits to the receiving antennas 4 in-vivo information including image data obtained by capturing the images of inside of the subject 2. The capsule endoscope 3 also has an antenna formed by a circular coil or a circular loop therein. When being swallowed into the subject 2, the capsule endoscope 3 passes through the esophagus in the subject 2 and moves through the body cavity due to the peristaltic motion of the gastrointestinal lumen. The capsule endoscope 3 captures sequentially images of the body cavity of the subject 2 at short-time intervals, for example, every 0.5 second, to generate image data of the captured images of inside of the subject 2, and transmits the same in sequence to the receiving antennas 4. In this case, the capsule endoscope 3 generates a transmission signal containing the image data and received strength detection data including positional information (beacon) for facilitating the detection of a received strength and the like, and transmits wirelessly a wireless signal obtained by modulating the generated transmission signal to the receiving antennas 4.

The receiving antennas 4 receive the wireless signal from the capsule endoscope 3 and transmit the wireless signal to the receiving device 6 via an antenna cable. The receiving antennas 4 are detachably connected to the antenna holder 5 at predetermined positions. The receiving antennas 4 are each composed of a loop antenna, an antenna pad for storing the loop antenna, and the like. Hook and loop fasteners are provided on one surface of the receiving antennas 4. In the following explanation, the number of the receiving antennas is three for the sake of simplicity. However, the number of the receiving antennas 4 is not limited to three.

The antenna holder 5 has a belt-shaped sheet portion 51 that is wrapped around the body surface of the subject 2, and shape detection units 52 that are provided on an outer surface 51a of the sheet portion 51 along a longitudinal direction of the sheet portion 51 to detect the shape of the sheet portion 51. The sheet portion 51 has on the outer surface 51a indexes M1 indicating the positon of the sheet portion 51. The sheet portion 51 also has a positioning hole 511 for positioning the antenna holder 5 relative to the naval of the subject 2. The detailed configuration of the antenna holder 5 will be described later.

The receiving device 6 records the image data on the inside of the subject 2 based on the wireless signal transmitted from the capsule endoscope 3 via the receiving antennas 4. The receiving device 6 records positional information, time information indicating time, and others in a memory, in association with the received image data. The receiving device 6 is stored in a receiving device holder (not illustrated) and carried by the subject 2 while the capsule endoscope 3 captures images, for example, while the receiving device 6 is introduced from the mouth of the subject 2, passes through the digestive tract, and is discharged from the inside of the subject 2. After the end of the inspection by the capsule endoscope 3, the receiving device 6 is removed from the subject 2 and is connected to the image processing device 7 for transmission of the image data and the like received from the capsule endoscope 3. The receiving device 6 also includes a display unit 63 that displays images corresponding to the image data received from the capsule endoscope 3 via the receiving antennas 4 or the position where the receiving antennas 4 are to be connected to the antenna holder 5.

The image processing device 7 displays the images corresponding to the image data on the inside of the subject 2 acquired via the receiving device 6. The image processing device 7 includes a cradle 7a that reads image data from the memory of the receiving device 6 and operation input devices 7b such as a keyboard and a mouse. When being attached to the receiving device 6, the cradle 7a acquires from the memory of the receiving device 6 the image data, the received strength information associated with the image data, and related information such as the time information and identification information for the capsule endoscope 3, and then transfers the acquired various kinds of information to the image processing device 7. The operation input device 7b accepts user input. The user observes the body parts of inside of the subject 2, for example, the esophagus, the stomach, the small intestine, the large intestine, and others while operating the operation input devices 7b and watching the images of inside of the subject 2 displayed in sequence by the image processing device 7, and diagnoses the condition of the subject 2.

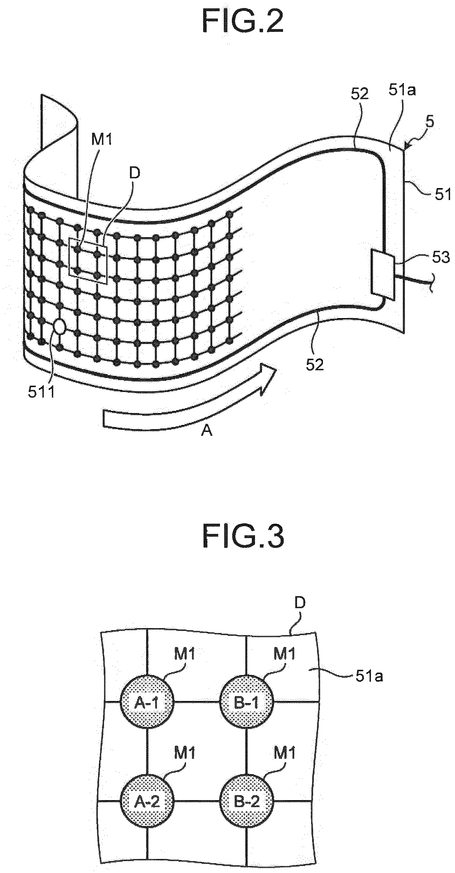

Next, a configuration of the antenna holder 5 illustrated in FIG. 1 will be explained. FIG. 2 is a perspective view of a general configuration of the antenna holder 5.

The antenna holder 5 illustrated in FIG. 2 has the sheet portion 51 that is wrapped around and attached on the body surface of the subject 2, the shape detection units 52 that are provided on the outer surface 51a of the sheet portion 51 along the longitudinal direction of the sheet portion 51 to detect the shape of the sheet portion 51, and an I/F unit 53 for connection between the shape detection units 52 and the receiving device 6.

The sheet portion 51 is composed of an elastic material and has the hook and loop fastener on the outer surface 51a. The sheet portion 51 also has the hook and loop fasteners near both end portions in the longitudinal direction (arrow A). This allows the sheet portion 51 to be wrapped around and attached on the trunk of the subject 2 regardless of the body shape of the subject 2.

The sheet portion 51 has the plurality of indexes M1 indicating the positions on the outer surface 51a. Specifically, the indexes M1 are provided on the outer surface 51a of the sheet portion 51 at predetermined intervals in an orthogonal coordinate system in which an X axis is set along the longitudinal direction of the sheet portion 51 and a Y axis is set along a short side direction of the sheet portion 51. FIG. 3 is an enlarged view of a region D illustrated in FIG. 2. As illustrated in FIG. 3, the sheet portion 51 has the indexes M1 at predetermined intervals. Each of the indexes M1 has printed information indicating the position on the sheet portion 51, for example, the character A for the X coordinate and the character 1 for the Y coordinate.

The shape detection units 52 are provided on the outer surface 51a of the sheet portion 51 along the longitudinal direction of the sheet portion 51 (arrow A). Specifically, the shape detection units 52 are provided on the outer surface 51a in the direction of wrapping the sheet portion 51 around the subject 2. The shape detection units 52 are provided at least near edge portions in a width direction (short side direction) of the sheet portion 51. Specifically, the shape detection unit 52 is provided between the edge portion in the width direction of the sheet portion 51 and the indexes M1. The shape detection units 52 are composed of shape sensors as three-dimensional shape detection sensors using optical fiber and light-emitting elements. The shape sensor is a sensor by which light is passed through a laminated linear special fiber loop to detect a bend in a target subject taking advantage of the fact that the bend increases or diminishes depending on a bend in the light. The shape detection units 52 are electrically connected to the receiving device 6 via the I/F unit 53. In the following explanation, the number of the shape detection units 52 is two but the number of the shape detection units 52 is not limited to two. In addition, the positions of the shape detection units 52 on the outer surface 51a of the sheet portion 51 can be changed as appropriate. Further, the shape detection units 52 may not only be provided on the outer surface 51a of the sheet portion 51 but may be provided on the inner surface of the sheet portion 51 or built into the sheet portion 51. Instead of the shape sensors, the shape detection units 52 may be used as an appropriate combination of an optical sensor, a magnetic sensor, a gyro sensor, and an acceleration sensor.

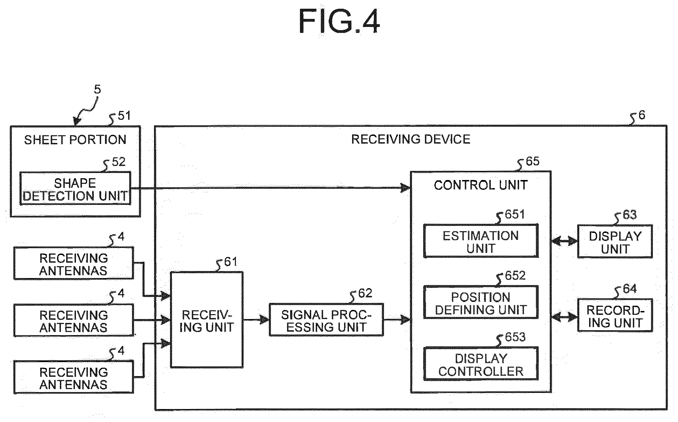

Next, a functional configuration of the receiving device 6 illustrated in FIG. 1 will be explained. FIG. 4 is a block diagram illustrating the functional configuration of the receiving device 6.

As illustrated in FIG. 4, the receiving device 6 includes a receiving unit 61, a signal processing unit 62, the display unit 63, a recording unit 64, and a control unit 65.

The receiving unit 61 performs processing on the wireless signal received via the receiving antennas 4 such as demodulation or amplification, for example, and outputs the results to the signal processing unit 62.

The signal processing unit 62 extracts the image data from the wireless signal input from the receiving unit 61, and performs predetermined processing, for example, various kinds of image processing and A/D conversion processing on the extracted image data, and outputs the results to the control unit 65. In this example, the image processing includes amplification and noise reduction of the image data.

The display unit 63 displays images corresponding to the image data input from the signal processing unit 62 under control of the control unit 65. The display unit 63 also displays index information indicating a plurality of indexes corresponding to the attachment positions of the plurality of antennas 4 on the outer surface 51a of the sheet portion 51 under control of the control unit 65. The display unit 63 is composed of a display panel of organic EL (electro luminescence), liquid crystal, or the like.

The recording unit 64 records the image data captured by the capsule endoscope 3 and various kinds of information associated with the image data, for example, positional information for the capsule endoscope 3 and identification information for identifying the receiving antennas 4 having received the wireless signal. In addition, the recording unit 64 records various programs executed by the receiving device 6 and data being processed. The recording unit 64 is composed of a recording medium such as a flash memory, a RAM (random access memory), or a memory card.

The control unit 65 performs centralized control on the operations of the components constituting the receiving device 6. The control unit 65 is composed of a CPU (central processing unit). The control unit 65 has an estimation unit 651, a position defining unit 652, and a display controller 653.

The estimation unit 651 estimates the cross-sectional shape of the trunk of the subject 2 based on the shape of the sheet portion 51 detected by the shape detection units 52. For example, the estimation unit 651 refers to a plurality of model patterns indicating human skeletons or body shapes recorded in the recording unit 64 to estimate the cross-sectional shape of the trunk of the subject 2 according to the shape of the sheet portion 51 detected by the shape detection units 52.

The position defining unit 652 decides the optimum attachment positions of the receiving antennas on the subject 2 based on the cross-sectional shape of the trunk of the subject 2 estimated by the estimation unit 651, and defines the plurality of indexes M1 corresponding to the attachment positions of the plurality of receiving antennas 4 on the outer surface 51a of the sheet portion 51 corresponding to the attachment positions.

The display controller 653 displays on the display unit 63 index information indicating the indexes corresponding to the attachment positions of the plurality of receiving antennas 4 on the sheet portion 51 defined by the position defining unit 652. Specifically, the display controller 653 displays, as the index information, information for specifying the positions of the indexes M1 on the outer surface 51a of the sheet portion 51.

A procedure for connecting the plurality of receiving antennas 4 to the sheet portion 51 with the use of the antenna holder 5 and the receiving device 6 configured as described above will be explained.

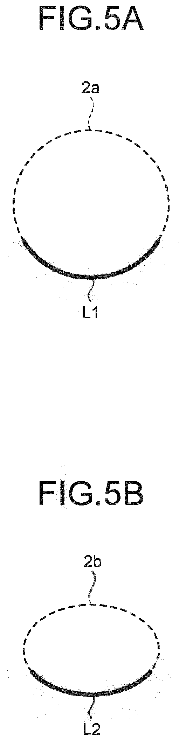

First, the antenna holder 5 is wrapped around and attached on the trunk of the subject 2. Then, the shape detection units 52 detect the shape of the sheet portion 51. Specifically, as illustrated in FIG. 5A or 5B, the shape detection units 52 detect a change in the attachment shape of the antenna holder 5 in response to the cross-sectional shape of the trunk of a subject 2a or a subject 2b. In FIGS. 5A and 5B, a thick line L1 and a thick curve line L2 corresponding to the shape detection units 52 indicate the shape of the sheet portion 51, and dotted lines indicate the trunks of the subject 2a and the subject 2b. After that, the shape detection units 52 output information on the shape of the sheet portion 51 to the receiving device 6 via the I/F unit 53.

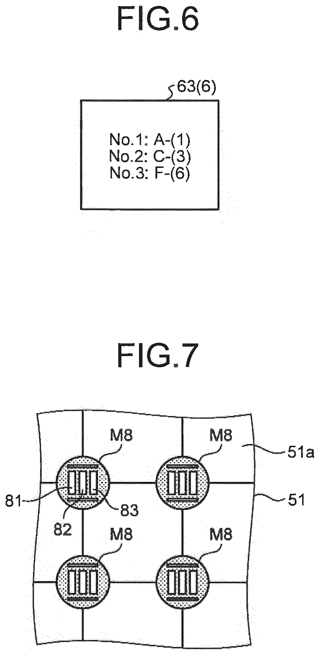

Subsequently, the estimation unit 651 estimates the cross-sectional shape of the truck of the subject 2 based on the shape of the sheet portion 51 input from the shape detection units 52. After that, the position defining unit 652 defines the indexes corresponding to the attachment positions of the plurality of receiving antennas 4 on the sheet portion 51 based on the cross-sectional shape of the trunk of the subject 2 estimated by the estimation unit 651. Then, the display controller 653 displays on the display unit 63 the index information corresponding to the attachment positions of the plurality of receiving antennas 4 on the sheet portion 51 defined by the position defining unit 652. Specifically, as illustrated in FIG. 6, the display controller 653 displays on the display unit 63 the index information indicating the indexes defining the attachment positions of the plurality of receiving antennas 4 by the indexes M1 of the sheet portion 51. After that, the operator connects the receiving antennas 4 to the indexes M1 at the specified positions according to the indication on the display unit 63.

According to one embodiment of the present invention described above, the receiving antennas 4 are attached to the outer surface 51a of the sheet portion 51 of the antenna holder 5 at the specified positions in accordance with the indication on the display unit 63. This makes it easy to attach the receiving antennas 4 to the antenna holder 5 at the predetermined receiving positions regardless of the body shape of the subject 2.

### (First Modified Example)

In a first modified example according to the embodiment of the present invention, instead of the coordinates as the indexes on the sheet portion 51, the outer surface 51a of the sheet portion 51 may be divided into a plurality of areas at predetermined intervals such that the areas are colored in identifiable manners and used as indexes. For example, the outer surface 51a of the sheet portion 51 may be divided into predetermined areas such that different colors are applied to the individual areas. In this case, the display controller 653 displays by the colors on the display unit 63 the index information corresponding to the attachment positions of the plurality of receiving antennas 4 on the sheet portion 51 defined by the position defining unit 652.

### (Second Modified Example)

In a second modified example according to the embodiment of the present invention, instead of the coordinates on the outer surface 51a of the sheet portion 51, light-emitting units for emitting light of predetermined colors may be provided at predetermined intervals. FIG. 7 is a schematic view of a configuration of the light-emitting units according to the second modified example of the embodiment of the present invention.

As illustrated in FIG. 7, light-emitting units M8 are provided on the sheet portion 51 at predetermined intervals. The light-emitting units M8 emit light of specific colors from among a plurality of preset colors. Specifically, each of the light-emitting units M8 is provided in a package of three red, green, and blue LEDs (light emitting diodes) 81, 82, and 83 that can independently emit light. The light-emitting units M8 emit light of seven colors, red, green, blue, yellow, magenta, cyan, and white by changing timings for light emission of the three color LEDs. The light-emitting units M8 are connected to the receiving device 6.

In this case, the display controller 653 causes the light-emitting units M8 corresponding to the attachment positions of the plurality of receiving antennas 4 on the outer surface 51a of the sheet portion 51 defined by the position defining unit 652 to emit light of specific colors, and displays on the display unit 63 the colors of the light-emitting units M8 as the index information indicating the indexes corresponding to the attachment positions of the plurality of receiving antennas 4 on the outer surface 51a of the sheet portion 51 defined by the position defining unit 652 in association with the receiving antennas 4. This allows the user to grasp the attachment positions of the receiving antennas 4 by intuition. Instead of the colors of the light-emitting units M8, the receiving antennas 4 may be assigned colors in advance. This makes it easy to attach the receiving antennas 4 even if the display unit 63 of the receiving device 6 is invisible.

In the foregoing embodiment, the estimation unit and the position defining unit are provided in the receiving device. Alternatively, the estimation unit and the position defining unit may be provided on the sheet portion of the antenna holder, for example.

### Reference Signs List

- 1: Capsule endoscope system

- 2, 2a, 2b: Subject
- 3: Capsule endoscope
- 4: Receiving antenna
- 5: Antenna holder
- 6: Receiving device
- 7: Image processing device
- M8: Light-emitting unit
- 51: Sheet portion
- 52: Shape detection unit
- 53: I/F unit
- 61: Receiving unit
- 62: Signal processing unit
- 63: Display unit
- 64: Recording unit
- 65: Control unit
- 511: Positioning hole
- M1: Index

## Claims

1. An antenna receiving device comprising:
a belt-shaped sheet portion to which a plurality of receiving antennas is detachably attached to receive information transmitted from a body-insertable apparatus introduced into a subject to acquire in-vivo information of the subject, the sheet portion being configured to be wrapped around and attached on a trunk of the subject;
a plurality of indexes provided on an outer surface of the sheet portion to indicate positions on the outer surface;
shape detection units provided on the outer surface of the sheet portion along a longitudinal direction of the sheet portion to detect a shape of the sheet portion;
an estimation unit configured to estimate a cross-sectional shape of the trunk based on the shape of the sheet portion detected by the shape detection units;
a position defining unit configured to define the plurality of indexes corresponding to attachment positions of the plurality of receiving antennas on the outer surface of the sheet portion based on the cross-sectional shape estimated by the estimation unit; and
a display unit configured to display index information indicating the plurality of indexes defined by the position defining unit.

2. The antenna receiving device according to claim 1, wherein the shape detection units are respectively provided at least near edge portions in a width direction of the sheet portion.

3. The antenna receiving device according to claim 1, wherein
the plurality of indexes is coordinates indicating the positions on the outer surface of the sheet portion, and
the display unit is configured to display the index information by the coordinates.

4. The antenna receiving device according to claim 1, wherein
the outer surface of the sheet portion is divided into a plurality of areas at predetermined intervals such that different colors are applied to the areas as the indexes, and
the display unit is configured to display the index information by the colors.

5. The antenna receiving device according to claim 1, wherein
the plurality of indexes is light-emitting units configured to emit light of predetermined colors, and
the display unit is configured to display the plurality of indexes by the colors of the light emitted from the light-emitting units.

6. An antenna holder configured to be connected to a receiving device for displaying images corresponding to information that is transmitted from a body-insertable apparatus introduced into a subject to acquire in-vivo information of the subject and that is received by a plurality of receiving antennas, the antenna holder comprising:
a belt-shaped sheet portion to which the plurality of receiving antennas is detachably attached, the sheet portion being configured to be wrapped around and attached on a trunk of the subject;
a plurality of indexes provided on an outer surface of the sheet portion to indicate positions on the outer surface; and
a shape detection unit provided on the outer surface of the sheet portion along a longitudinal direction of the sheet portion to detect a shape of the sheet portion.

7. A receiving device for displaying information that is transmitted from a body-insertable apparatus introduced into a subject to acquire in-vivo information of the subject and that is received by a plurality of receiving antennas, the receiving device comprising:
an estimation unit configured to estimate a cross-sectional shape of a trunk of the subject based on a shape of a belt-shaped sheet portion wrapped around and attached on the trunk of the subject, the shape of the sheet portion has been detected by a shape detection unit provided along a longitudinal direction of the sheet portion;
a position defining unit configured to define, based on the cross-sectional shape estimated by the estimation unit, a plurality of indexes provided on an outer surface of the sheet portion to indicate positions on the sheet portion, the plurality of indexes corresponding to attachment positions of the plurality of receiving antennas on the outer surface of the sheet portion; and
a display unit configured to display index information indicating the plurality of indexes defined by the position defining unit.
